(19) Europäisches Patentamt
European Patent Office
Office européen des brevets

(11) **EP 1 976 480 B1**

(12) **EUROPEAN PATENT SPECIFICATION**

(45) Date of publication and mention
of the grant of the patent:
**17.05.2017 Bulletin 2017/20**

(51) Int Cl.:
***A61Q 19/10*** (2006.01)   ***A61K 8/02*** (2006.01)
***A61K 8/81*** (2006.01)   ***A61Q 11/00*** (2006.01)

(21) Application number: **06846779.4**

(22) Date of filing: **22.12.2006**

(86) International application number:
**PCT/US2006/062539**

(87) International publication number:
**WO 2007/076466 (05.07.2007 Gazette 2007/27)**

(54) **FILM CONTAINING COMPOSITIONS**

FILMHALTIGE ZUSAMMENSETZUNGEN

COMPOSITIONS CONTENANT UN FILM

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **22.12.2005 US 316626**

(43) Date of publication of application:
**08.10.2008 Bulletin 2008/41**

(73) Proprietor: **Colgate-Palmolive Company
New York, NY 10022 (US)**

(72) Inventors:
 • **OMER, Mohamed
  Piscataway, NJ 08854 (US)**
 • **IBRAHIM, Sayed
  Somerset, NJ 08873 (US)**
 • **CONNOR, Kevin
  Toms River, NJ 08755 (US)**

 • **SALKO, Diane
  Cream Ridge, NJ 08514 (US)**
 • **HASKEL, Ariel
  East Brunswick, NJ 08816 (US)**
 • **WISNIEWSKI, Karen Lee
  Bound Brook, NJ 08805 (US)**

(74) Representative: **Wichmann, Hendrik et al
Wuesthoff & Wuesthoff
Patentanwälte PartG mbB
Schweigerstraße 2
81541 München (DE)**

(56) References cited:
EP-A- 1 153 594      WO-A-01/17488
WO-A-99/22710      WO-A-2005/110344
WO-A-2006/013081    US-A1- 2002 051 797
US-A1- 2002 110 536   US-A1- 2002 169 270
US-A1- 2004 086 468   US-A1- 2006 018 845
US-A1- 2006 292 088

**Description**

BACKGROUND OF THE INVENTION

Compositions for enhancing health, hygiene, or appearance, such as oral care compositions, skin care compositions, and hair care compositions, are used by millions of people. These compositions are used for a wide variety of purposes, including to enhance personal health, hygiene, and appearance, as well as for preventing or treating a variety of diseases and other conditions in humans and in animals. The formulation of such compositions presents a number of challenges. They must be pharmaceutically and/or cosmetically acceptable for their intended use. Compositions that contain therapeutic agents preferably deliver the therapeutic agents at effective levels, avoiding undue chemical degradation during storage or use. Similarly, compositions containing cosmetically functional materials must deliver the material at effective levels under the conditions that they are typically used by the consumer.

The aesthetic appeal of all such compositions is also important and can have significant effects on consumer acceptance and usage. The art seeks to further improve the aesthetic effects as well as the cosmetic and therapeutic benefits of these products. Indeed, many such compositions known in the art are deficient in one or more attributes. Thus, there is an ongoing need for new cleansing products and personal and oral care compositions, and methods of their use.

SUMMARY OF THE INVENTION

The present invention provides a composition according to claim 1. Preferred features are defined in the dependent claims. In various embodiments, the invention provides compositions comprising a film entrained in a carrier (or continuous medium) in which the film comprises at least two polymers and optionally a functional material. One of the polymers is more soluble in water than the other polymer, one of the polymers being substantially water-soluble and the other polymer being substantially water-insoluble. The compositions may be useful for oral care, personal care or household cleansing, including dentifrice compositions, hand and body cleansing, shampoos and skin and hair conditioners and lotions compositions, liquid dish cleansers, fabric detergents and softeners, and hard surface cleansers.

The compositions of the invention may contain various carriers (aqueous or non-aqueous), additives, excipients, and adjuvants, depending on the end use of the compositions. As described herein, all amounts are provided by weight percent of the total composition, unless otherwise specified.

DETAILED DESCRIPTION OF THE INVENTION

The compositions of the invention have a carrier containing a film. The film may be present as a plurality of flakes, fragments, or pieces, which may be of a desired shape and thickness. The film includes at least one functional material and a combination of at least two polymers, one of which is more water-soluble than the other. In compositions of the present invention, the film includes a water-soluble polymer and a water-insoluble polymer. In various embodiments, "water-soluble" refers to a polymer that dissolves in water at 20°C. A first polymer "more soluble in water" than a second polymer means that the second polymer may not dissolve in water, may only partially dissolve in water or dissolve to a lesser extent compared to the first polymer, or may require a higher water temperature and/or a longer time (other test conditions being equal) to dissolve in water.

As referred to herein, a "film" is a material having a substantially lamellar structure. A "lamellar" structure has, or is capable of having, a size in one or two dimensions (e.g., the x- or y-dimensions) that is substantially greater than the thickness of the structure in a third dimension (e.g., the z-direction). Lamellar structures among those useful herein include those that are substantially planar, layered, or lamelliform. In one embodiment, the lamellar structure is substantially planar, having a size in both the x- and y-dimensions that is substantially greater than the z-direction. In other embodiments, the lamellar structure is non-planar. In one embodiment, a film of this invention comprises a substantially continuous surface that can appear as a substantially flat surface, although in some embodiments the film may be deformed. In such embodiments, the film can have any of a number of shapes, including having a smooth curved surface. The fragments may be of a desired size and may be of regular or irregular perimeter.

The film contains at least two polymers, one of which is more soluble in water than the other. In certain embodiments, the film has at least one water-soluble polymer and one water-insoluble polymer.

The functional material may be one that enhances the aesthetic appeal of the compositions, such as a colorant or texturing agent, or may be an active agent. The functional materials include therapeutic active materials, flavorants, colorants, cosmetic materials, and fragrances. In certain embodiments, the film includes a combination of substantially water-soluble and substantially water-insoluble polymers. The polymers of the film are selected and apportioned to provide a desired stability and/or rate of disintegration during use of the composition and/or to provide a desired rate of release or delivery of a functional material during use of the composition. In certain embodiments, the polymers of the film are selected and apportioned to release an active agent that contributes to the efficacy of the composition during use. In certain embodiments, the film comprises a weight ratio of water-soluble polymer to water-insoluble polymer of about 20 to 1 to about 1 to 10.

In various embodiments, the present invention provides aqueous compositions containing a plurality of film pieces, flakes, or fragments. The film pieces, flakes, or fragments comprise a functional material, a combination of water-soluble and water-insoluble polymers, and, optionally, a water-soluble filler material. In various embodiments, the functional material may be selected from therapeutic active materials, flavorants, colorants, pearlescent materials, beads, cosmetic materials, and fragrances.

In various embodiments, the film thickness, polymer combination, and, optionally, water-soluble filler content may be adjusted to provide one or more of these features: (1) a desired stability of the film in the composition, (2) a desired rate of disintegration of the film during use of the composition, and (3) a desired rate of delivery of a functional material during use of the composition. An increased level of the film's water-soluble components may increase the rate of disintegration of the film, particularly with combined with a shear force imposed on the film during use of the aqueous composition. An increased level of the water-insoluble components may increase stability of the film in the aqueous composition. The relative amounts of water-insoluble polymer and water-soluble polymer may be such that the film flakes disintegrate during use of the aqueous composition to release the active agent. The relative amounts of water-insoluble polymer, water-soluble polymer and, optionally, water-soluble filler may be selected to release an amount of active agent proportional to how vigorously or how long the aqueous composition is used, e.g., by brushing, scrubbing, or rubbing action during use of the aqueous composition.

The carrier may be hydrophilic or hydrophobic. In certain aspects of the invention, the aqueous carrier has a yield value of 3 to 20 Pascal (Pa) and a viscosity of 2,000 to 20,000 centipoise (cP). In certain embodiments of the invention, the carrier is aqueous and thixotropic and has a viscosity of at least about 2,000 centipoise.

As is known in the art, the carrier may be formulated to contain one or more surfactant materials. These materials may be selected and/or formulated to provide to the composition any structure known or to be developed in the art, e.g., a structure that is lamellar, spherulite, micellar, and/or extended micellar.

In addition, the carrier may contain substantial amounts of a hydrophobic phase. The amount of water or aqueous phase present in the carrier relative to the amount of hydrophobic materials of the hydrophobic phase may be about 1:1 by weight to about 10:0.01 by weight. The hydrophobic phase may be present in any physical structure, e.g., it may be emulsified or suspended.

Disclosed herein is a method of making a cleansing, oral or personal care composition containing a film formulated with at least two polymers, one polymer being more soluble in water than the other. The film also comprises a functional material so that the flakes are substantially stable in the composition but release the functional material at a desired rate when the composition is used for its intended purpose.

Also disclosed herein are methods for making a functional material available for administration to a human or animal subject by oral or topical application of a composition comprising a film entrained in a carrier, wherein the film comprises the functional material. Also disclosed herein are methods for making a functional material available for cleansing or treatment of a surface by application of a composition comprising a film entrained in a carrier, wherein the film comprises the functional material. In each case, the film may be present as a plurality of fragments, pieces, or flakes. The functional material may be provided by disruption or disintegration of the film. The film has a combination of polymers, one of which is water soluble and another that is less water soluble (and may be water-insoluble), that provides a desired rate of release with continued application of disrupting or disintegrating force.

As is known in the art, the water solubility of a given polymer will vary depending on the chemical structure of the polymer and of the type and relative ratios of monomers that make up the homo- or copolymer. Thus, the structure of the first polymer and the second polymer of the invention can be varied to the advantage of the end use. Useful polymers (homo- and co-) that can be used to include, for example and without limitation. poly(vinyl pyrrolidone) [PVP], which may have a weight average molecular weight of about 100,000 or more and up to about 1.5 million, poly(vinyl alcohol) [PVA], poly(vinyl pyrrolidone/vinyl acetate) copolymers, e.g., 60:40 by weight vinyl pyrrolidone : vinyl acetate. ethylene oxide graft copolymers of PVA, water-soluble cellulose derivatives including hydroxylated and carboxylated celluloses including hydroxyalkyl cellulose and hydroxyalkyl alkyl cellulose polymers such as hydroxypropyl methyl cellulose (HPMC), hydroxypropyl cellulose (HPC) and hydroxyethyl cellulose (HEC); polyethylene oxide polymers, and gum arabic.

Other polymers (homo- and co-) include, for example and without limitation, acrylic copolymers including those that are those that are hydrophobically modified (which may be in the form of acrylic copolymer dispersions), crosslinked poly(vinyl pyrrolidone). poly(vinyl acetate) [PVAc], certain cellulose derivatives such as cellulose acetate, cellulose nitrate, alkyl cellulose such as ethyl cellulose, butyl cellulose, and isopropyl cellulose, cellulose acetate phthalate, unneutralized carboxymethyl cellulose shellac, ethylene-vinyl acetate copolymers, silicone polymer (e.g., dimethylsilicone), polyesters, polyurethanes, nylons, gums such as xanthan and carrageenan, polyethylene, and polypropylene. Acrylic copolymers may include carboxylic acid functionality which has not been neutralized at all or not been sufficiently neutralized to render the copolymer water soluble. Preferred acrylic copolymers are film-forming polymers. In certain embodiments, the acrylic copolymers may have weight average molecular weights at least about 20,000, more preferably at least about 50,000, and up to about 1,000,000, more preferably up to about 900,000. An example of a suitable, film-forming acrylic copolymer is a tert-butyl acrylate/ethyl acrylate/methacrylic acid copolymer. The water-insoluble polymers may be prepared as dispersions (e.g., by emulsion polymerization) and may be stabilized with suitable emulsifiers. One useful PVAc emulsion, for example, is a 30 weight % dispersion of PVAc in water stabilized with 2.7 weight % PVP and 0.3% sodium lauryl sulfate. An example of an acrylic copolymer dispersion is a 30% by weight aqueous dispersion of an ethyl acrylate: methyl methacrylate copolymer (weight ratio of ethyl acrylate to methyl methacrylate approximately 2 to 1).

In some embodiments, the film may include a partially water-soluble polymer in addition to a water-insoluble polymer. Examples of partially water-soluble polymers include, without limitation, poly(vinyl pyrrolidone/vinyl acetate) copolymers in which the weight % of vinyl pyrrolidone monomer is 60% by weight or greater and physical mixtures of PVP and PVAc.

In a hydrophilic or aqueous composition, the relative amounts of water-soluble polymer and water-insoluble and/or partially water-soluble polymer in the film may be selected such that the film is storage-stable but disintegrate during use of the composition. In various embodiments, the film may include at least 0.1 % by weight, at least about 5% by weight, or at least about 10% by weight water-soluble polymer, and may include up to about 90 weight %, up to about 80 weight %, or up to about 50 weight % of water-soluble polymer. In various embodiments, the film may include at least 0.1% by weight, at least about 1% by weight, or at least about 9% by weight water-insoluble polymer, and may include up to about 80 weight %, up to about 70 weight %, up to about 60 weight %, or up to about 40 weight % of water-insoluble polymer. In addition to or instead of the water-insoluble polymer(s), the film may include one or more partially water-soluble or water-swellable polymers in amounts of generally about 0.1 to about 70 weight %, or about 0.1 to about 50 weight %, or about 1 to about 10 weight %. In various embodiments, a method of stabilizing film fragments in an aqueous cleanser, personal care, or oral care composition uses water-soluble and water-insoluble materials in the film that are balanced for stability in the composition but deliver of active ingredient when the composition is used.

In compositions of the present invention, a combination of PVP and water-soluble poly(vinyl pyrrolidone/vinyl acetate) copolymer is used as the water-soluble polymer. The relative amounts of PVP and water-soluble poly(vinyl pyrrolidone/vinyl acetate) copolymer may be about 0.1:10 to about 1:0.1 by weight, or about 0.5:10 to about 1:0.5 by weight. In compositions of the present invention, the water-insoluble polymer comprises one or more polyacrylate copolymers, for example, which can be combined with the PVP and water-soluble poly(vinyl pyrrolidone/vinyl acetate) copolymer.

The film may further include a water-soluble filler. Suitable examples of water-soluble fillers include, without limitation, materials including natural gums such as sodium alginate, carrageenan, xanthan gum, gum acacia, arabic gum, guar gum, pullulan, agar, chitin, chitosan, pectin, karaya gum, zein, hordein, gliadin, locust bean gum, tragacanth and other polysaccharides; starches such as maltodextrin, amylose, high amylose starch, corn starch, potato starch, rice starch, tapioca starch, pea starch, sweet potato starch, barley starch, wheat starch, waxy corn starch, modified starch (e.g., hydroxypropylated high amylose starch), dextrin, levan, elsinan and gluten; and proteins such as collagen, whey protein isolate, casein, milk protein, soy protein and gelatin. The film may further include a dispersible or swellable filler such as one or more of modified starches, alginate esters, and divalent or multivalent ion salts of alginates.

In certain embodiments, the film includes at least one functional material, which is described in detail in the following section. Other desired materials may be included in the film, such as, without limitation, surfactants, emulsifiers, plasticizers such as oils and polyols including mineral oil, glycerol, and propylene glycol, clays, inert starch particles, cellulose, or other fillers, plastigels, waxes, texture modifiers such as cold water swellable, physically modified, and pregelatinized starches.

The film preferably contains at least one functional material. A "functional material" is a material providing aesthetic or decorative effects to the composition or having a desired utility in the composition when used for its intended purpose. In various embodiments, such utilities provided during use may be therapeutic, cosmetic, aesthetic, decorative, sensory or the functional material may enhance the performance of the composition during its intended use. The functional material may have a combination of utilities. In some embodiments, the film can comprise a plurality of functional materials. In one embodiment, the compositions of the present invention comprise a plurality of first film fragments having a first functional material, and a plurality of second film fragments having a second functional material, wherein the second functional material differs or is distinct from the first functional material. In another embodiment, the compositions of the present invention comprise a plurality of layered film fragments having a first functional material in a first layer of the film and a second functional material different from the first functional material in a second layer of the film.

In various embodiments, the functional materials may have utilities are therapeutic, cosmetic, aesthetic, decorative, sensory, performance-enhancing, or combinations thereof. The functional material is preferably selected according to the kind of composition the film will be used in.

In one example useful for many types of compositions, the film pieces may include one or more colorants. Colorants may be pigments or dyes, including metallic and pearlescent pigments, and may be introduced into the film as a solid or as a color concentrate (*e.g.*, a dye-containing. particulate polyethylene). In various embodiments, the film comprises a formulation colorant that imparts a color to the film. In various embodiments, the film fragments contrast with the carrier, and are white, black, or of any color that is visible against or contrasts with the carrier of the composition. Any colorants well known in the art are suitable for use in the compositions of the invention. Formulation colorants among those useful herein include non-toxic water soluble dyes or pigments, such as, for example, metallic oxide "lakes." In certain embodiments, the colorant is approved for incorporation into a food or drug by a regulatory agency. such as FD&C or D&C pigments and dyes approved by the FDA for use in the United States. In one embodiment, the colorant comprises a water insoluble inorganic pigment, such as titanium dioxide. chromium oxide green, phthalocyanine green, ultramarine blue, ferric oxide, metallic pigments such as aluminum flake pigments, pearlescent pigments such as pearlescent mica pigments, or a water insoluble dye lake. In some embodiments, dye lakes include calcium or aluminum salts of an FD&C dye such as FD&C Green #1 lake, FD&C Blue #2 lake, D&C Red #30 lake or FD&C Yellow #15 lake. In certain embodiments, a water soluble dye, such as, for example, FD&C Blue #1 is contained within a water-insoluble polymer such as, for example polyethylene such as that found in polyethylene beads. In certain embodiments, the film comprises a dye such as D&C Red #30. In certain embodiments, a white colorant is used, for example titanium dioxide ($TiO_2$). titanium dioxide coated mica (*e.g.*, Timiron), a mineral, or a clay. The carrier containing the colored film may contrast with the film or be transparent so that preferably the film can be seen through the carrier.

In certain embodiments, the colorant is a non-bleeding dye. In various embodiments, the film comprises a colorant at a level of about 0.5% to about 20% by weight of the film, or about 1% to about 15% by weight of the film, or about 3% to about 12% by weight of the film. In one embodiment, the compositions of the present invention comprise a first plurality of film fragments comprising a first color, and a second plurality of film fragments comprising a second color. Preferably, the second color is different than the first color and/or the shape of the first plurality of film fragments is different from the shape of the second plurality of film fragments. In other words, the compositions may include two kinds of film fragments that differ in color, shape, or both color and shape.

In some embodiments, color space coordinates of phases of a composition, such as, for example, a film and a carrier (e.g.. toothpaste), can be determined separately. In certain embodiments, the coordinates for a product film/carrier pairing can be quite far apart (such as disclosed in Example 12, infra), and can contribute to a compositions aesthetic appeal, for example by contributing to a striking nature of a composition's aesthetic appeal. In certain alternative embodiments, the coordinates for a product film/carrier pairing can be not particularly large yet still have a noticeable aesthetic effect. In certain embodiments, the L a* b* system established by the Commission Internationale d'Eclairage (CIE) is used to establish color values. (See, for example. McClelland, D., Macworld® Photoshop® 4 Bible, IDG Books Worldwide. Inc. 1997, pp. 157-184.) In addition. the quantity $\Delta E^*$ can also be indicative of noticeable color differences. $\Delta E^*$ can be determined using the following equation.

$$\Delta E^* = \{ (\Delta L^*)^2 (\Delta a^*)^2 + (\Delta b^*)^2 \}^{1/2}$$

where $\Delta L^*$ is the difference in lightness, and $\Delta a^*$ and $\Delta b^*$ are the differences in the color space coordinates, $a^*$ and $b^*$. In certain configurations, color value measurements can be made using a chromameter, with data collection in the $L^*a^*b^*$ color coordinate mode using standard procedures.

In various embodiments, for example for a toothpaste composition, the functional material may comprise a flavorant. For example, in certain oral care embodiments a flavorant may be rapidly released as the fragments disintegrate during use of the product, delivering a breath freshening flavor or desired mouthfeel or sweetness into the mouth. Useful flavorants include, without limitation, synthetic flavor oils or a flavoring aromatics. oleo resins and extracts derived from plants, leaves, flowers, fruits and so forth, and combinations thereof. Representative flavor oils include spearmint oil, cinnamon oil, peppermint oil, clove oil. bay oil, thyme oil, cedar leaf oil, oil of nutmeg. oil of sage, and oil of bitter almonds. Flavorants can be used individually or in combination. Commonly used flavors include mints such as peppermint, artificial vanilla, cinnamon oil, and various fruit flavors. In certain embodiments, the film comprises flavoring or food additive, such as those described in Chemicals Used in Food Processing, publication 1274 by the National Academy of Sciences, pages 63-258. In various embodiments, the film comprises a flavorant at a level of about 1% to about 30% by weight of the film, or about 8% to about 25% by weight of the film.

In various embodiments, the film also comprises a sweetener. Sweeteners among those useful herein include natural and synthetic sweeteners. In one embodiment, the sweetener is a water soluble sweetening agent such as a monosaccharide, a disaccharide or a polysaccharide. For example, water soluble sweetening agents include xylose, ribose, glucose (dextrose), mannose. lactose, fructose (levulose), sucrose (sugar), maltose, a soluble saccharin salt, *i.e.,* a sodium or a calcium saccharin salt, a cyclamate salt. dipeptide based sweeteners, such an L-aspartic acid derived sweetener such as L-aspartyl-L-phenylanaline methyl ester (aspartame). In various embodiments, the film comprises a sweetener at a level of about 0.01% to about 10% by weight of the film.

In some embodiments, flavorants and sweeteners may be combined in one or more films or film layers to allow flavorant or sweetener blooming, where the character of the composition taste changes as the films or film layers are being used.

In various embodiments, such as personal care, dish cleanser, fabric detergent, fabric conditioner, and hard surface cleanser compositions, the functional material comprises a fragrance, which may be incorporated into the film or encapsulated and the encapsulated fragrance incorporated into the film. In one embodiment, fragrances may be layered in the film to allow fragrance blooming, where the character of the fragrance changes as the different layers of the film are being used.

In some embodiments, for example for personal cleansing and skin-care compositions. the functional material may comprise a material for skin protection and/or a sunscreens, including ultraviolet light absorbers (UV absorbers) such as those listed by the CTFA. for example cinnamates-based light absorbers, benzene-based (*i.e.,* avobenzone, oxybenzone) based light absorbers, para-amino benzoic acid and its derivatives, and benzophenone-type UV absorbers. The UV absorber or absorbers may be present in the film in an amount of at least about 0.001 weight %, or at least about 0.01 weight %, and up to about 25 weight %, or up to about 13 weight %.

In various embodiments, the film comprises a therapeutic active. As referred to herein, a therapeutic active is a material that is useful for the prevention or treatment of a physiological disorder. condition, or systemic disease. Such disorders or conditions include those of the oral cavity (including the teeth and gingiva), skin, and hair. The specific therapeutic active is preferably determined according to the desired utility of the composition. Such actives include the following.

A. antimicrobial agents and preservatives, such as triclosan, parabens such as methyl paraben, potassium methyl paraben, potassium butyl paraben, potassium phenyl paraben, and sodium isobutyl paraben; cetyl pyridinium chloride, domiphen bromide, quaternary ammonium salts, zinc compounds, octonidine, EDTA, essential oils such as thymol, methyl salicylate, eucalyptol and menthol, and the like,

B. non-steroidal anti-inflammatory drugs, such as aspirin, acetaminophen, ibuprofen, ketoprofen, diflunisal, fenoprofen calcium, naproxen, tolmetin sodium, indomethacin, and the like,

C. anti-tussives, such as benzonatate, caramiphen edisylate, menthol, dextromethorphan hydrobromide, chlophedianol hydrochloride, and the like,

D. decongestants, such as pseudoephedrine hydrochloride, phenylepherine, phenylpropanolamine, pseudoephedrine sulfate, and the like,

E. anti-histamines, such as brompheniramine maleate, chlorpheniramine maleate, carbinoxamine maleate, clemastine fumarate, dexchlorpheniramine maleate, diphenhydramine hydrochloride, diphenylpyraline hydrochloride, azatadine maleate, diphenhydramine citrate, doxylamine succinate, promethazine hydrochloride, pyrilamine maleate, tripelennamine citrate, triprolidine hydrochloride, acrivastine, loratadine, brompheniramine, dexbrompheniramine, and the like,

F. expectorants, such as guaifenesin, ipecac, potassium iodide, terpin hydrate, and the like,

G. anti-diarrheals, such a loperamide, and the like,

H. $H_2$-antagonists, such as famotidine, ranitidine, and the like; and

I. proton pump inhibitors, such as omeprazole, lansoprazole, and the like,

J. general nonselective CNS depressants, such as aliphatic alcohols, barbiturates and the like,

K. general nonselective CNS stimulants such as caffeine, nicotine, strychnine, picrotoxin, pentylenetetrazol and the like,

L. drugs that selectively modify CNS function such as phenyhydantoin, phenobarbital, primidone, carbamazepine, ethosuximide, methsuximide, phensuximide, trimethadione, diazepam, benzodiazepines, phenacemide, pheneturide, acetazolamide, sulthiame, bromide, and the like,

M. antiparkinsonism drugs such as levodopa, amantadine and the like,

N. narcotic-analgesics such as morphine, heroin, hydromorphone, metopon. oxymorphone, levorphanol, codeine, hydrocodone, xycodone, nalorphine, naloxone, naltrexone and the like,

O. analgesic-antipyretics such as salicylates, phenylbutazone, indomethacin, phenacetin and the like,

P. psychopharmacological drugs such as chlorpromazine, methotrimeprazine. haloperidol, clozapine, reserpine, imipramine, tranylcypromine, phenelzine, lithium and the like.

The amount of medicament that can be used in the films can be dependent upon the dose needed to provide an effective amount of the therapeutic active.

In various embodiments, the film comprises a preservative. A preservative can be added in amounts of about 0.001 wt % to about 5 wt %, preferably of about 0.01 wt % to about 1 wt % of the film. Non-limiting examples of preservatives include sodium benzoate and potassium sorbate.

The films may contain other functional materials, depending on the specific intended use of the composition. In particular, in certain oral care embodiments, the film comprises an oral care active, which is useful for the prevention or treatment of an oral care disorder or condition. Oral care actives among those useful herein include: tartar control agents such as dialkali or tetraalkali metal pyrophosphate salts, long chain polyphosphates such as sodium hexametaphosphate; and cyclic phosphates such as sodium trimetaphosphate; antibacterial agents such as magnolia extract, triclosan, grapeseed extract, thymol, methyl salicylate, eucalyptol, menthol, hop acids, cetyl pyridinium chloride, (including CPC/Zn and CPC + enzymes) and ursnic acid; zinc ion sources such as zinc gluconate, zinc citrate, zinc chlorite and alpha ionone; tooth desensitizers such as potassium nitrate, desensitizing polymers, and desensitizing minerals; anti-inflammatory agents such as magnolia extract: vitamins such as panthenol, retinyl palmitate, folic acid, tocopherol acetate and Vitamin A; herbs or herbal extracts such as rosemary, oregano, chamomilla recutita, mentha piperita. salvia officinalis, orcommiphora and myrrha; proteins, such as milk proteins and enzymes such as peroxide-producing enzymes, amylase, plaque-disrupting agents such as papain, glucoamylase, glucose oxidase, and "next generation" enzymes; whitening agents such as hydrogen peroxide, urea peroxide and phosphate salts; medicinals, such as aspirin (acetyl salicylic acid), caffeine, and benzocaine; probiotics; abrasives such as silicas (including high cleaning silica); anti-caries agents such as stannous ion sources (e.g., stannous fluoride) or amino fluoride; nitric oxide synthase inhibitors such as guanidinoethyldisulfide; calcium; antiattachment ingredients, such as polyumylphosphonic acid and N$\alpha$-acyl amino acid alkyl esters and salts, more particularly, N$\alpha$-acyl arginine alkyl esters such as ELAH; silicones; chlorophyll compounds, anti-leukoplakia agents such as beta-carotene; antioxidants such as Vitamin E; and combinations thereof. Active materials among those useful herein are described in U.S. Pat. No. 6,596,298, Leung et al. In some embodiments, the films comprise such active materials at a concentration of about 0.01 to about 30% by weight of film, of about 2% to about 25% by weight of the film, or of about 10% to about 20% by weight of film.

In various personal care compositions, useful therapeutic agents include conditioning agents including skin or hair conditioners, moisturizers, humectants, including naturally and non-naturally present humectants, skin conditioners, hair conditioners, antiperspirant actives, deodorant actives, anesthetics, antibacterial agents, anti-inflammatory agents, vitamins, proteins, skin lipid fluidizers, enzymes and other proteins, vitamins, occlusive agents, amino acids, exfoliants including both physical and chemical exfoliants, skin whiteners, anti-aging additives, ingredients to treat conditions of the skin (for example acne or psoriasis), and mixtures thereof.

In certain skin care embodiments (e.g., lotions), the functional material comprises a material selected from the group consisting of surfactants, conditioning agents, moisturizers, sunscreens, UV absorbers, antioxidants, enzymes and other proteins, vitamins, antibacterial agents, odor reducing agents, steroids, anti-inflammatory agents, naturally and non-naturally occurring humectants, skin lipid fluidizers, occlusive agents, amino acids, physical and chemical exfoliants, skin whiteners, anti-aging, antiperspirant actives, and mixtures thereof.

In certain hair care embodiments (e.g., shampoos and conditioners), the functional material comprises a material selected from the group consisting of surfactants, colorants, denaturants, film forming polymers, conditioning agents, fixatives, hair color stabilizers, antiinflammatoy agents, antioxidants, moisturizers, enzymes and other proteins, vitamins, antidandruff agents, sunscreen agents, and mixtures thereof. Suitable conditioning agents include silicones (e.g., silicone oils, cationic silicones, silicone gums, high refractive silicones, and silicone resins), and organic conditioning oils (e.g., hydrocarbon oils, polyolefins, and fatty esters). Suitable anti-dandruff agents include: pyridinethione salts. selenium sulfide, particulate sulfur, and mixtures thereof. Suitable anti-inflammatories include glucocorticoids and nonsteroidal anti-inflammatories. Suitable colorants include non-oxidative dyes such as "direct action dyes," metallic dyes, metal chelate dyes, fibre reactive dyes and other synthetic and natural dyes. Suitable sunscreen agents include 2-ethylhexyl p-methoxycinnamate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylme- thane. 4-isopropyl dibenzoylmethane. 3-benzylidene camphor, 3-(4-methylbenzylidene) camphor, titanium dioxide, zinc oxide, silica, iron oxide, and mixtures thereof. Suitable vitamins include Vitamin E and panthenol.

In certain antiperspirant or deodorant embodiments, the functional material comprises a material selected from the group consisting of fragrances, alcohols, antimicrobial agents, antiperspirant salts. odor reducing agents. moisturizers, other components as described above for skin and hair care compositions, and mixtures thereof. Suitable antimicrobial agents include the primary oleamine salt of piroctone, certain metal salts of piroctone acid (such as aluminum, sodium, potassium, zirconium, calcium and zinc metal salts), triclosan, zinc phenolsulfonate, certain heavy metal salts of 1-hydroxy pyridinethione (such as zinc pyrithione, magnesium pyrithione, and aluminum pyrithione) and bacteriostatic quaternary ammonium compounds (such as cetyl-trimethyl ammonium bromide, cetyl pyridinium chloride, benzethonium chloride, and sodium N-lauryl-sarcosine), and carbonate and bicarbonate salts. Antiperspirant salts include polyvalent metal salts and complexes thereof, such as aluminum halides, aluminum hydroxy-halides, zirconyl oxyhalides, zirconyl hydroxy-halides, zinc compounds such as zinc phenylsulfonate, zinc glycinate and zinc pyrithione. Such complexes may include amino acids (e.g., glycine) forming antiperspirant actives commonly known as "ZAG," comprising aluminum. zirconium and chlorine having an Al:Zr ratio of about 1.67 to about 12.5 and a Metal:Cl ratio of about 0.73 to about 1.93. Odor reducing agents useful herein include sulfur precipitating agents such as copper gluconate, zinc citrate and zinc gluconate.

In certain personal hand and body cleansing and conditioning compositions (e.g., liquid soaps and bar soaps) the functional material comprises a material selected from the group consisting of fragrances, essential oils, emulsifying agents, thickening agents. colorants, surfactants, natural actives, therapeutic actives, stain prevention actives, antimicrobial agents, vitamins, natural extracts, amino acids, enzymes or other proteins, abrasives, odor control agents, conditioning agents, moisturizers, humectants, occlusive agents, skin lipid fluidizers. lipophilic actives, hydrophilic materials, pearlizers, opacifying agents, and combinations thereof, including such materials as described above.

In liquid dish cleanser compositions, the film may include a functional material selected from the group consisting of moisturizers, vitamins, abrasives such as silica, antimicrobials, therapeutic agents for treating skin conditions or other conditions that affect hands, and colorants. Liquid dish and gel liquid dish cleansing products may include films containing functional materials such as cleansing boosters, essential oils, emulsifying agents, thickening agents, colorants, surfactants, natural actives, therapeutic actives, anti-microbial agents, vitamins, natural extracts, amino acids, enzymes or other proteins, moisturizers, humectants, occlusive agents, skin lipid fluidizers, lipophilic actives, hydrophilic materials, pearlizers. opacifying agents, and combinations thereof.

In fabric care compositions (*e.g.*, detergents and fabric conditioners), the film may include functional materials selected from the group consisting of enzymes such as proteases, soil release agents, whiteners, antimicrobials, odor reducing agents, and fabric softening agents.

In various embodiments, the film comprises a compatibility enhanced active. As referred to herein, a "compatibility enhanced active" is a functional material that has enhanced utility in a composition wherein the material is a component of a film, relative to the utility of the material in a composition wherein the material is a component of the carrier. Such enhanced utility may be due to any of a variety of factors, including enhanced delivery or reduced physical or chemical degradation of the material. In some embodiments, the compatibility enhanced material is incompatible with a component or components of the carrier. A component which is incompatible with a carrier can be, for example, a component which reacts chemically or forms a precipitate with a component of the carrier.

Compatibility enhanced actives among those useful in oral compositions, for example. include cationic antimicrobials, calcium salts, fluoride salts, enzymes and other proteins, and other ingredients incompatible with anionic components, and mixtures thereof. In one dentifrice embodiment, a compatibility enhanced active is a cationic antimicrobial. such an antimicrobial comprising a quaternary group. Examples of such antimicrobials include cetyl pyridinium chloride (CPC), chlorhexidine, and ethyllauroylarginine HCl. Such actives are generally incompatible with carriers comprising the surfactant sodium lauryl sulfate (SLS, a common component of dentifrices) or silicate abrasives. For example, in such a composition, the CPC and SLS typically form a complex upon mixing. The formation of the complex renders both compounds ineffective for their intended purposes in a dentifrice. However, incorporation of CPC in a film in a dentifrice composition of this invention comprising both film and a carrier which comprises SLS will maintain both substances in the dentifrice in an effective state. Other examples of compatibility enhanced actives useful in dentifrice compositions of this invention include the anti-caries agent sodium fluoride (NaF), which is a component of some dentifrices, can be incompatible with calcium, which is also comprised by some dentifrices, because calcium fluoride ($CaF_2$), can form a precipitate. However. if one of these components (for example, sodium fluoride) is comprised by film fragments in an oral care composition, the composition can provide effective amounts of both calcium and the anti-caries agent. In personal care embodiments compatibility enhanced actives include, for example, moisturizers, conditioning agents, sunscreens, fragrances and any active ingredient insoluble within the carrier that does not create one physical phase, change the carrier's color, or produce haziness in the carrier.

The films of the present invention may be made in a variety of ways, including methods among those known in the art for making films. In various embodiments, the film may be prepared by forming a slurry of the film materials. The slurry may be an aqueous slurry and may contain suitable organic solvents as well, such as ethanol and propylene glycol, or it may be a nonaqueous slurry in an organic liquid such as ethanol, isopropanol, methanol, propylene glycol, and so on. The slurry may, for example, be a slurry containing 10-75% water and/or ethanol. Polymers in solid form (*e.g.*, powders or flakes) may be dissolved or slurried first in a portion of the water or ethanol and then mixed with other film ingredients.

The slurry is cast, drawn down, roll-coated, or otherwise applied in a layer on a substrate and dried to form a sheet of film material. The sheet can then be separated or released from the substrate. In one embodiment, the substrate material has a surface tension that allows the film slurry to spread substantially uniformly across the substrate surface, thereby avoiding formation of a destructive bond between the film and the substrate. Non-limiting examples of suitable substrates include glass, stainless steel, TEFLON® brand PTFE, sold by E.I. du Pont de Nemours of Wilmington, Delaware, and polyethylene- or silicone-impregnated or -coated paper. Following casting, the film is then dried.

Drying of the slurry can be carried out at elevated temperature, for example at a temperature of 60 to 100°C, with the aid of a drying oven, a drying terminal, a vacuum drier, or any other suitable drying equipment known in the art. If low boiling point solvents are used, the film can be dried at room temperature. The drying time will depend, among other factors, upon the amount and type of liquid components and film thickness. The film may retain residual solvent (e.g., residual water). In other embodiments, the film is made by extrusion of the film composition through a die, followed by cutting to a desired thickness, and drying. In other embodiments, the film is made by solvent casting. Shapes may be cut or punched from the film, or the film may fragmented in a different manner to form fragments, flakes, or pieces of film that are incorporated into the carrier.

In one embodiment, the film comprises more than one layer. For example, a film can comprise a first layer comprising a polymer, and one or more additional layers that provide a coating. The coating can be, for example, a shellac coating. A coating can comprise a layer on either or both sides of a polymer layer. Thus, in some embodiments a process for making a multi-layered film can comprise forming a first layer of a film, such as a polymer layer, then coating the first layer with a second or subsequent layer of a coating material, for example shellac. The film may also be a multi-layer film having one or more layers of equal or unequal thicknesses. At least one layer includes a functional material. In some embodiments, each layer may include a functional material, which may be the same or different.

In various particular embodiments, the film may be formed from a slurry including:

> 10-75% by weight ethanol
> 0.1-80%, preferably 5-50% by weight of a polypyrrolidone/vinyl acetate copolymer (60:40)
> 0.1-90%, preferably 9-50% by weight of a PVP homopolymer
> 1-60%, preferably 9-40% by weight of a 30% by weight of a 30% by weight aqueous dispersion of a methyl methacrylate copolymer (ratio of ethyl acrylate to methyl methacrylate is about 2 to 1)
> 0-30% by weight of a 30% by weight solution of a *tert*-butyl acrylate/ethyl acrylate/methacrylic acid copolymer
> 0-50% by weight of a PVAc emulsion (30 % by weight dispersion of PVAc with 2.7% by weight PVP, 0.3% sodium lauryl sulfate)
> 0.1-30% by weight of a colorant
> 0.1-10% by weight beads
> (e.g., exfoliating, abrasive, pearlescent, shimmer)
> 0-40% by weight fragrance and/or flavor
> 0-20% by weight mineral oil or other oil, and
> 0-5% by weight UV absorber or other active agent.

In various embodiments, the film pieces exhibit perceivable contrast with the carrier. The perceivable contrast can be sensory contrast, such as optical contrast, tactile contrast, taste contrast, or olfactory contrast. In some configurations, optical contrast can be color contrast, or a difference in refractive index or reflective index. In some configurations, color contrast can be imparted by one or more colorants that comprise different components of the composition. In various embodiments, the present invention provides compositions comprising a plurality of film fragments in a carrier, wherein said fragments are visibly discernable. As referred to herein, "visibly discernable" refers to one or more characteristics of a fragment which cause the fragment to have a different physical appearance, preferably to the naked eye, relative to the carrier in which the fragment is entrained. Such characteristics include color, opacity, refractive index. reflective index, size, shape. and combinations thereof.

In various embodiments, the film pieces have a non-random shape. In one embodiment, a "non-random" shape is a shape which results from a manufacturing process of shaping, cutting, or other forming process by which a specific shape is imparted to a fragment. In such embodiments, a non-random shape is distinguished from such shapes that result from simple precipitation or grinding of a material. In one embodiment, a "non-random" shape is "repeating," wherein the composition comprises a plurality of film pieces have substantially the same shape. Such repeating shape may have any of a variety of forms, and may be selected based on a variety of aesthetic or functional criteria. In certain embodiments, the shape of a film fragment can be a recognizable shape. In certain embodiments, a film fragment can comprise a nonrandom shape. Such shapes include simple geometric shapes. having a fragment perimeter of polygons and elliptical shapes, such as triangles, quadrilaterals (such as a square, a rectangle. a rhombus), pentagons, hexagons, ovals. and circles. In one embodiment, the repeating shape is a square. Repeating shapes include, in other embodiments, shapes that are representative of figures or animate or inanimate objects. such as stars, hearts, gems, flowers, trees, shamrocks, a letter of an alphabet, numbers, animals, people, and faces. In various embodiments, the composition comprises a single repeating shape. In other embodiments, the composition comprises a plurality of fragments having a plurality of repeating shapes. In one embodiment, the compositions of the present invention comprise a plurality of first film fragments having a first repeated shape, color, and/or functional material and a plurality of second film fragments having a second, different repeated shape, color. and/or functional material.

The appearance of the film fragments, including their shape, color, and texture, may be suggestive-that is, may provide a visual cue-for a characteristic of a product into which they are placed or of the product itself. Thus, the appearance of the film fragments may suggest the presence of a certain active of benefit, an environmental benefit, an ingredient or class of ingredients including therapeutic materials and fragrances, or a general concept such as relaxation, anti-aging, whitening or cleaning, or health. For example a citrus fragrance may be incorporated into film pieces having citric fruits-like shape, a moisturizer or cream may be incorporated into film shaped as white drops, or an impression of outdoors or a "forest" may be suggested by tree-shaped film pieces.

A preferred film thickness for a particular application depends upon considerations such as the amount of water in the carrier and the desired stability of the film in the carrier, the use for which the composition comprising carrier and film is intended, the desired rate of release of an active during use of the composition, and so on. In certain embodiments, such as for a personal cleansing composition (e.g., body wash or liquid hand soap) or cleaning composition such as dish soap, with a water content of 50-90 weight % and a surfactant content of 1-20 weight %, the film has a thickness of preferably at least about 0.001 mm, more preferably at least about 0.05 mm, and up to about 0.8 mm, more preferably up to about 0.3 mm. In various embodiments, the size of the fragments is not critical, and may be determined pursuant to any of a variety of criteria, including manufacturing convenience, affect on visual appearance, surface area, affect on texture in the composition, and combinations thereof. In some embodiments, the film fragments can be up to about 1 inch (25.4 mm) in length in the longest dimension, but more typically the film fragments may be up to about 3.2mm (1/8 inch) along their longest dimension. The "long dimension" is the longer dimension of a fragment in length or width (i.e., in the x-and y-dimensions, as the fragment is, or is deformed to be, in a planar shape) in a dimension substantially perpendicular to the "thickness" or shortest dimension of the fragment (i.e., the z-dimension). It is understood that in various embodiments comprising a plurality of film pieces, the pieces may be present in a range of sizes due to a variety of factors, including random variation in size, manufacturing tolerances, and intentional sizing or mixing of the fragments through sieving or other means. Sizes refer to the average size of fragments in a given plurality of pieces.

In one embodiment, wherein the film fragment comprises mother-of-pearl or a pearlescent pigment, the fragments are greater than about 590 microns in their longest dimension. In one embodiment, wherein the fragment comprises pearlescent pigments of mica film fragments coated with a thin layer of titanium dioxide, the film fragments are greater than 110 microns in their longest dimension.

In some embodiments, the compositions of the present invention comprise film pieces having an aspect ratio of at least about 5:1. "Aspect ratio" of a film piece is the ratio of the diameter of the smallest imaginary sphere that can enclose the object to the diameter of the largest imaginary sphere that can be completely inside the object and tangent to the surfaces of the object. For example, the aspect ratio of a sphere is 1:1: in another example, the aspect ratio of a cylinder that is 2 inches (50.8 mm) long and 1/4 inch (6.35 mm) in diameter is slightly over 8:1; in yet another example, a film piece of the present invention that is 1 mil (25.4 microns) in thickness, 1 inch (25.4 mm) in length, and 1 inch (25.4 mm) wide has an aspect ratio of about 1414:1.

In some embodiments, the compositions of the present invention comprise film pieces having an aspect ratio of at least about 10:1. In various embodiments, the film pieces have an aspect ratio of about 5:1 to about 10.000:1, about 10: 1 to about 1,000:1, or about 20:1 to about 100:1, or about 25:1 to about 35:1.

In various embodiments, the film comprises a first plurality of pieces and a second plurality of pieces, wherein the first plurality of pieces differ in composition or appearance from the second plurality of pieces. Such difference in composition or appearance can be in any aspect of the composition of the film pieces (e.g., different film components, different functional material, different formulation colorant), different appearance (e.g., shape, color, texture, refractive index, reflective index), or combinations thereof.

The compositions of the present invention comprise a carrier in which a film or film pieces are entrained. A "carrier" is any material or composition in which the film can be entrained. In various embodiments comprising a plurality of film pieces, such film pieces may be entrained by embedding, suspension, dispersion or other distribution of the film pieces in the carrier. In various embodiments, the film pieces are distributed substantially homogenously throughout the carrier. In other embodiments, the film pieces are not distributed homogenously in the carrier. In certain embodiments, the distribution of a plurality of film pieces is substantially isotropic within the carrier.

In various embodiments, the carrier is a liquid, semi-solid or solid. A liquid can be a thixotropic liquid or can be a Newtonian liquid. A "semi-solid" as used herein can be a gel, a colloid, or a gum. As used herein, semi-solids and liquids are fluids distinguished on the basis of viscosity: a semi-solid is a high viscosity fluid, while a liquid has lower viscosity. There is no definitive dividing line between these two types of fluids. A semi-solid can, in certain embodiments, have a viscosity as high as thousands of Pa·s. Carriers among those useful herein include liquids, pastes, ointments, and gels, and can be transparent, translucent or opaque.

In certain embodiments, the composition comprising film pieces can be a skin care composition, for example, a soap, a lotion, a body wash, a skin conditioner, a bath gel, a shampoo, a conditioner, a deodorant, an antiperspirant, a fragrance, a perfume, a cosmetic or combinations thereof, such as an antiperspirant/deodorant. In certain embodiments, a composition comprising at least two phases can be a hair care composition, such as, for example, a shampoo or a conditioner, or a combination thereof.

The specific composition of the carrier preferably depends on the intended use of the composition. In various embodiments, the carrier is aqueous, comprising about 5% to about 95% water. The aqueous carrier includes water and may include one or more organic solvents, preferably water-miscible organic solvents. In other embodiments, the carrier is substantially non-aqueous.

The carrier may comprise any of a variety of materials, including emulsifiers, thickeners, fillers, and preservatives. In some embodiments, the carrier comprises a functional material, such as those described above. In some embodiments, the carrier comprises the same functional material as the film.

In certain embodiments, the compositions of the present invention are oral care compositions suitable for administration to the oral cavity. Such compositions include dentifrices, mouthwashes, dental gels, lozenges, beads, gums, oral strips, mints, liquid toothpastes, sprays, paint-on gels, lip balms, whitening strips, breath strips, oral chews, and combinations thereof. An oral care composition disclosed herein can be used, for example, for cavity prevention, whitening, plaque prevention or reduction, gingivitis prevention or reduction, tartar control, sensitivity prevention or reduction, or breath malodor prevention or reduction, and stain prevention.

In various embodiments, an orally acceptable dentifrice carrier may include water, viscosity modifiers, diluents, thickeners, gelling agents, surfactants, emulsifiers, foam modulators, pH modifying agents, abrasives, polishing agents such as colloidal silica and alkali metal aluminosilicate complexes, humectants, mouth feel agents, sweetening agents, flavor agents, colorants, preservatives, anticaries agents, tartar control agents, antibacterial agents, anti-inflammatory agents, antioxidants, and combinations thereof. The carrier may, for example, comprise a humectant such as glycerin, sorbitol or an alkylene glycol at a level of about 10% to about 80% by weight, or about 20% to about 60% by weight of the composition.

In various embodiments, the dentifrice compositions contain relatively low amounts of water. In one embodiment, the compositions contain less than 10% by weight water, for example less than 8% by weight or less than 6% by weight water. In various embodiments, dentifrice compositions contain at least one humectant, useful for example to prevent hardening of a toothpaste upon exposure to air. Any orally acceptable humectant can be used, including without limitation polyhydric alcohols such as glycerin, propylene glycol, sorbitol, xylitol and low molecular weight polyethylene glycol (PEG). One or more humectants are optionally present in a total amount of about 1% to about 70%, for example about 1% to about 50%, about 2% to about 25%, or about 5% to about 15% by weight of the composition.

In one embodiment a dentifrice composition of the invention comprises at least one abrasive, such as, without limitation silica, for example in the form of silica gel, hydrated silica or precipitated silica, alumina, insoluble phosphates, calcium carbonate, resinous abrasives such as urea-formaldehyde condensation products and the like. One or more abrasives are optionally present in an abrasive effective total amount, typically about 5% to about 70%, for example about 10% to about 50% or about 15% to about 30% by weight of the composition. Average particle size of an abrasive, if present, is generally about 0.1 to about 30 microns, for example about 1 to about 20 microns or about 5 to about 15 microns.

In various embodiments a composition of the invention comprises at least one surfactant, useful for example to compatibilize other components of the composition and thereby provide enhanced stability, to help in cleaning the dental surface through detergency, and to provide foam upon agitation, *e.g.*, during brushing with a dentifrice composition of the invention. Any orally acceptable surfactant, most of which are anionic, nonionic or amphoteric, can be used. Suitable anionic surfactants include without limitation water-soluble salts of $C_{8-20}$ alkyl sulfates, sulfonated monoglycerides of $C_{8-20}$ fatty acids, sarcosinates, taurates and the like. Illustrative examples of these and other classes include sodium lauryl sulfate, sodium coconut monoglyceride sulfonate, sodium lauryl sarcosinate, sodium lauryl isoethionate, sodium laureth carboxylate and sodium dodecyl benzenesulfonale. Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation derivatives of $C_{8-20}$ aliphatic secondary and tertiary amines having an anionic group such as carboxylate, sulfate, sulfonate, phosphate or phosphonate. A suitable example is cocoamidopropyl betaine. One or more surfactants are optionally present in a total amount of about 0.01% to about 10%, for example about 0.05% to about 5% or about 0.1% to about 2% by weight of the composition.

The oral compositions optionally contain other active ingredients. Non-limiting examples include desensitizing agents, stannous ion sources, zinc ion sources, sialagogues, breath-freshening agents, antiplaque agents, anti-inflammatory agents additional to any anti-inflammatory phenolic compound present, periodontal agents, anti-gingivitis agents, analgesics and nutrients. Actives should be selected for compatibility with each other and with other ingredients of the composition. The oral compositions optionally contain one or more other non-active ingredients. Non-limiting examples include diluents, bicarbonate salts, pH modifying agents, foam modulators, thickening agents, viscosity modifiers, pigmenting agents, sweeteners, flavorants and colorants. Tooth pastes, tooth gels, and similar dentifrice compositions are formulated with these and optionally other additives according to known principles.

In some embodiments, the carrier containing the film pieces may be suitable for use as a body cleansing formulation, such as, for example, a liquid hand cleanser, a liquid body cleanser, a bar soap, or a soap-based gel formulation. In these embodiments, a carrier can comprise a cleansing formulation such as, in non-limiting example, a polyethylene glycol diisostearate or a polyethylene glycol diisostearate having an average degree of ethoxylation of about 40-100.

The compositions of the present invention may comprise an aqueous body wash carrier in which a film or film pieces are entrained. A polymeric rheology modifier provides several properties such as a yield value, flow, viscosity, thickening, and suspending ability. For our purpose yield value also referred to as yield point is defined as the initial resistance to flow under stress. It can be measured using a constant stress rheometer Brookfield YR-1 Yield Rheometer using a #72 spindle at an appropriate rotational speed. In certain preferred embodiments, the yield point is at least about 3 Pascal, and may preferably be up to about 15 Pascal or up to 20 Pascal. The viscosity of the body wash carrier is preferably at least about 4,000 centipoise, and may be up to about 20,000 centipoise, preferably up to about 10,000 centipoise or up to about 15,000 centipoise as determined using a Brookfield DV -II+ Viscometer using a #5 spindle at an appropriate rotational speed (from 10-30 RPM).

In certain embodiments, a body wash carrier includes a polymeric rheology modifier that comprises a crosslinked, alkali-swellable, acrylate emulsion copolymer. One useful crosslinked, alkali-swellable acrylate emulsion copolymer is described in Schmucker-Castner et al., U.S. Pat. No. 6,635,702. The acrylate copolymer includes about 20%, more preferably about 35%, and up to about 80%, more preferably up to about 65% by weight of at least one carboxylic acid monomeric unit. The carboxylic acid monomeric unit may be obtained by copolymerizing at least one ethylenically unsaturated, carboxylic acid-functional or anhydride-functional monomer. Suitable examples of such monomers include, without limitation, acrylic acid, methacrylic acid, crotonic acid, maleic acid, itaconic acid, fumaric acid, aconitic acid, anhydrides of these, half esters of these that are diacids, and combinations of any of these. The acrylate copolymer also includes a substantial amount of crosslinks. Crosslinking may be provided by copolymerization of a polyethylenically unsaturated monomer or by copolymerization of monoethylenically unsaturated monomers having mutually reactive functionalities (in addition to their unsaturation) that undergo reaction before, during, after the addition polymerization to provide the desired crosslinking. Suitable examples of polyethylenically unsaturated monomers include, without limitation, polyalkyenyl ethers of sucrose or others polyols; diallyl phthalates; divinyl benzene; allyl (meth)acrylate, trimethylolpropane tri(meth)acrylate, diallyl itaconate, diallyl fumarate, diallyl maleate, hexanediol di(meth)acrylate, butanediol di(meth)acrylate, neopentyl glycol di(meth)acrylate, pentaerythritol tetra(meth)acrylate, dipentaerythritol penta(meth)acrylate, dipentaerythritol hexa(meth)acrylate, alkylene glycol di(meth)acrylates and polyalkylene glycol di(meth)acrylates, such as ethylene glycol di(meth)acrylate, butylene glycol di(meth)acrylate, diethylene glycol di(meth)acrylate, triethylene glycol di(meth)acrylate, and polyethylene glycol di(meth)acrylate; diallyl terephthalate, and so on, as well as combinations of such monomers. A variety of pairs of mutually reactive groups are possible. Illustrative examples of such pairs of reactive groups include, without limitation, epoxide and carboxyl groups, amine and carboxyl groups, epoxide and amine groups, epoxide and anhydride groups, amine and anhydride groups, hydroxyl and carboxyl or anhydride groups, amine and acid chloride groups, alkylene-imine and carboxyl groups, organoalkoxysilane and carboxyl groups, isocyanate and hydroxyl groups, cyclic carbonate and amine groups, isocyanate and amine groups, and so on. Specific examples of such monomers include, without limitation, glycidyl (meth)acrylate with (meth)acrylic acid. N-alkoxymethylated acrylamides (which react with themselves) such as N-isobutoxymethylated acrylamide, gamma-methacryloxytrialkoxysilane (which reacts with itself); hydroxyalkyl (meth)acrylates or reactive amino acrylates (such as tert-butylamino ethyl methacrylate) along with polycaprolactone derivatives of these combined with carboxylic acid- or anhydride- or isocyanate-functional monomers; and combinations thereof. The polyethylenically unsaturated monomers or combinations of mutually reactive monomers may be at least about 0.01 %, preferably at least about 0.03%, and up to about 5%, preferably up to about 3% by weight of the monomers that are copolymerized. Other copolymerizable monomers that have no acid groups and are not crosslinking monomers may be used in desired amounts. Examples of suitable comonomers include, without limitation, esters of acrylic, methacrylic, and crotonic acids and diesters of $\alpha,\beta$-ethylenically unsaturated dicarboxylic acids containing 4 to 6 carbon atoms; vinyl esters, vinyl ethers, vinyl ketones, and aromatic or heterocyclic aliphatic vinyl compounds, such as methyl (meth)acrylate, ethyl (meth)acrylate, n-butyl (meth)acrylate, 2-ethylhexyl (meth)acrylate, hydroxyalkyl (meth)acrylates such as hydroxyethyl (meth)acrylate, vinyl acetate, styrene, vinyl chloride, vinylidene chloride, acrylonitrile, acrylamide, N,N-dimethylacrylamine, tert-butylacrylamide, and combinations thereof. In certain embodiments, the crosslinked, alkali-swellable acrylate copolymer includes at least about 35 weight % and up to about 65 weight % of such comonomers. The crosslinked, alkali-swellable acrylate copolymer may be prepared by emulsion polymerization.

The crosslinked, alkali-swellable acrylate copolymer may be included in the carrier in an amount of at least about 1%, preferably at least about 1.8%, and more preferably at least about 2.5% by weight of the carrier. Alternatively, the copolymer may be present in an amount of up to about 5% by weight. For example, when preparing a transparent composition, it may be preferred to use a rheology modifier that provides a carrier with a clarity of at least about 2 NTU and up to about 25 NTU, as determined, for example, using a Hach 2100P Turbidimeter.

The crosslinked, alkali-swellable acrylate copolymer is neutralized with an alkaline neutralizing agent. Alkaline neutralizing agents include inorganic and organic bases such as those selected from the group consisting of alkali hydroxides and alkanolamines, particularly, sodium hydroxide or triethanolamine.

The body wash carrier may further include one or more surfactants. Any known in the art are suitable, including anionic surfactants, amphoteric surfactants, quaternary surfactants, cationic surfactants, and combinations of these. Suitable examples of anionic surfactants include, without limitation, sulfuric acid derivatives, alkyl sulfates, ethoxylated alkyl sulfates such as sodium laureth sulfate and sodium pareth sulfate, ammonium lauryl sulfate, alkyl sulfonates, alkyl olefin sulfonates, alkylaryl sulfonates, alkyl succinates, alkyl sulfosuccinates, alkyl ethoxy sulfosuccinates, acylated amino acids and acyl peptides such acyl and alkyl glutamates, TEA lauroyl sarcosinate, potassium myristoyl hydrolyzed collagen; alkyl phosphates, alkyl ether carboxylates, alkyl isethionates, acyl amides, alkanoic acids, sodium ricinoleate, magnesium stearate, ester-functional and ether-functional carboxylic acids, sodium stearoyl lactylate, sodium trideceth-6 carboxylate, sulfonic acid derivatives such sodium methyl oleoyl taurate, sodium lauroyl isethionate, sodium dodecylbenzene sulfonate. sodium olefin sulfonates, and combinations of these. In certain embodiments the carrier includes preferably at least about 4%, more preferably at least about 7.5% by weight anionic surfactant and preferably up to about 12%, more preferably up to about 10% by weight anionic surfactant. Suitable examples of amphoteric surfactants include, without limitation, quaternary surfactants such as alkyl betaines, alkylamido betaines, alkyl sulfobetaines, alkyl sultaines and alkylamido sultaines, especially those having 8 to 18 carbons in the alkyl and acyl group, such as cocoamidopropyl betaine, and combinations of these. Other suitable examples of amphoteric surfactants include, without limitation, alkylamido alkyl amines such sodium capryloamphoacetate, lauroamphodipropionic acid; alkyl substituted amino acids such sodium lauriiminodipropionate, myristaminopropionic acid. In certain embodiments the carrier includes preferably at least about 0.5%, more preferably at least about 1.25% by weight anionic surfactant and preferably up to about 3%, more preferably up to about 1.75% by weight amphoteric surfactant. In certain instances, it is preferred to include at least one anionic surfactant and at least one amphoteric, cationic and quaternary, surfactant in the carrier.

In various embodiments, the carrier comprises at least about 50% by weight water or at least about 62% by weight water, and up to about 90% by weight water or up to about 80% by weight water.

The body wash carrier may be prepared by conventional mixing techniques. As an example, an acrylate copolymer emulsion may be added to water with agitation, then anionic surfactant(s) may be added and neutralized to a desired pH, followed by addition of amphoteric surfactant(s).

The body wash carrier may optionally include effective amounts of various other materials, for example colorants such as those mentioned above with regard to film functional materials, fragrances, antibacterials, preservatives, antioxidants, moisturizers, humectants, skin conditioning agents, anti-aging agents, fragrance beads, exfoliating beads, moisturizing beads, mica, glitter, opacifying agents, viscosity adjusters, pH adjusters, and pearlizing agents. In certain embodiments the carrier is clear, but when desired a pearlizing agent such as ethylene glycol distearate may be used in amounts such as 0.1 to 5 % by weight of the carrier. One particular embodiment comprises shea butter beads in the range of 100-1200 microns. Another particular embodiment comprises polyethylene beads in the size range of 200 to 1000 microns as an exfoliant (for example in an amount of 0.01-2 weight %), or larger polyethylene beads (250-2000 microns) in smaller amounts (for example, in an amount of 0.01-1 weight %).

The carrier pH may be adjusted with an organic acid such as citric acid to a pH of about 8 to about 4, preferably about 6.8 to about 5.0, more preferably about 5.5 to about 6.5.

In some embodiments, a composition comprising a carrier and a plurality of film pieces can be suitable for use as a cosmetic, such as, for example, a mascara formulation that is removable with soap and water. In these embodiments, the carrier can be a mascara formulation such as disclosed in U.S. Pat. No. 6,503,495, Alwattari et al. In certain configurations, these carrier compositions can comprise about 3% to about 60% water-insoluble polymeric material, about 2% to about 50% water-soluble, film-forming polymers, and about 0.05% to about 20.0% organophilic clays. In certain embodiments, these compositions can be fabricated in a multitude of forms, such as creams, pastes and solids. In some embodiments, a mascara carrier composition can comprise water-insoluble polymeric materials in an aqueous emulsion. In certain embodiments, water-insoluble polymeric materials can be aqueous emulsions or dispersions of polymeric materials comprising polymers. In some configurations, the polymers can comprise precursor monomers, mixtures of monomers, natural polymers and mixtures thereof. In some configurations, a polymeric material can also include water-insoluble polymeric materials. In certain configurations, a water-insoluble polymer can comprise about 3% to about 60%; about 4% to about 40% or about 5% to about 30% by weight of the composition. In non-limiting examples, a water-insoluble polymeric material can comprise monomers selected from the group consisting of aromatic vinyls, dienes, vinyl cyanides, vinyl halides, vinylidene halides, vinyl esters, olefins and their isomers, vinyl pyrrolidone, unsaturated carboxylic acids, alkyl esters of unsaturated carboxylic acids, hydroxy derivatives of alkyl esters of unsaturated carboxylic acids, amides of unsaturated carboxylic acids, amine derivatives of unsaturated carboxylic acids, glycidyl derivatives of alkyl esters of unsaturated carboxylic acids, olefinic diamines and isomers, aromatic diamines, terephthaloyl halides, olefinic polyols and mixtures thereof.

In some embodiments, the carrier can be suitable for use as an antiperspirant, a deodorant, or an antiperspirant/deodorant. Deodorant and antiperspirant products may be in any of several forms including, for example, creams, liquids, aerosol liquids solid sticks. Such carriers can comprise an antiperspirant active (as described above), a deodorant active, an odor reducing material, emollients (such as described above), structurants, colorants, perfumes, thickeners distributing agents, emulsifiers, bacteriostats, and fungistats.

In some embodiments, the carrier can be suitable for use as a shampoo or hair conditioner. Such carriers may comprise solvents (e.g., water), surfactants (e.g., anionic, non-ionic, cationic and amphoteric surfactants), thickeners; propellants; powders; fillers; plasticizers; lubricants; and emollients and humectants and other functional materials (as described above).

In some embodiments, the carrier can be suitable for use as a lotion, such as, for example, lotion disclosed in U.S. Pat. No. 6,352,701 to Scholz et al. In some configurations, a composition comprising a lotion carrier and a plurality of film pieces can maintain or improve the skin condition after multiple applications without noticeable slimy or abnormal feeling during post application hand washing. In some aspects, when used as a presurgical scrub replacement, a composition comprising a lotion carrier and a plurality of film pieces can achieve bacterial, fungal, and viral kill equal to or better than a traditional soap and water scrub in a shorter period of time while maintaining or improving the skin's natural barrier to microbial and chemical contaminants. In certain embodiments, a composition comprising a lotion carrier and a plurality of film pieces can provide a viscous composition which includes a high concentration of a lower alcohol but does not require a polymeric thickener to make the composition viscous. Further, in certain aspects, a composition comprising a lotion carrier and a plurality of film pieces can have a cosmetically elegant feel and may be dispensed as a lotion or as a foam.

A composition comprising a lotion carrier and a plurality of film pieces can comprise a lower alcohol and water in a weight ratio of about 15:85 to 100:0, between at least 0.5% and 8.0% by weight thickener system comprised of at least two emulsifiers, each emulsifier present in at least 0.05% by weight wherein the emulsifiers can be selected such that the composition free of auxiliary thickeners has a viscosity of at least 4,000 centipoise at 23 °C and wherein each emulsifier can be comprised of at least one hydrophobic group and at least one hydrophilic group, wherein: (i) the hydrophobic group can be comprised of an alkyl group of at least 16 carbon atoms; an alkenyl group of at least 16 carbon atoms; or an arylalkyl or an arylalkenyl group of at least 20 carbon atoms; and (ii) the hydrophilic group of at least one emulsifier can be comprised of an amide group having the structure --NHC(O)R''' or --C(O)NHR'''' where R''' can be hydrogen or an alkyl group of 1-10 carbon atoms optionally substituted in available positions by N; O, and S atoms; an ester group of short chain alcohols or acids (*e.g.*,

L=-C(O)OR' or --OC(O)R' where R' can be $C_1$-$C_4$ branched or straight chain alkyl optionally substituted in available positions by hydroxyl groups); a polyglucoside group having 1-10 glucose units; a polyglycerol ester group having 1-15 glycerol units, a secondary amine group; a tertiary amine group; a quaternary amine group; an anionic group such as a sulfate, a sulfonate group, a phosphate group, a phosphonate group, a carboxylate group, or a zwitterionic group.

In various other embodiments, the invention provides compositions for cleansing or treatment of an inanimate substrate. In particular, the carrier may be a liquid or gel liquid dish cleaning composition in which the film pieces are suspended. The film pieces are dissolved or broken down when the dish cleaning composition is sheared, for example by application to a dish by sponge or cloth, or by dilution in warm water. A dish cleaning carrier may contain more than 30 weight % of active surfactant. Typical liquid dish cleaning carriers contain such materials as water, thickening agents, surfactants, detergent boosters, alcohols, such as ethanol, salt, antibacterial agents, pH adjustors and other functional materials (as described above).

The present invention provides processes for making compositions comprising a carrier comprising a film. In various embodiments, the film comprises a plurality of pieces that are combined with a carrier. In some configurations, a carrier and a plurality of film pieces can be mixed. In some configurations, the mixing can comprise slow stirring. In one embodiment, the present invention provides a process for making a composition comprising a carrier having distributed therein a plurality of lamellar pieces, wherein said process comprises:

    (a) providing the carrier;
    (b) adding the lamellar pieces to the carrier to form a mixture; and
    (c) homogenizing the mixture.

As referred to herein, "homogenizing" refers to the admixture of the pieces and the carrier so as to attain a substantially homogeneous distribution of pieces in the carrier. It should be noted, however, that the resulting composition still retains two-phase composition. Homogenizing may be accomplished using any of a variety of conventional homogenizers.

In another method, the film is added to a component of the carrier (e.g., to a humectant for a dentifrice, aqueous premix, fragrance in personal care applications). The rest of the carrier is then made, and the mixture of film is then added to the carrier.

Also disclosed herein are methods for the administering a functional material to a human or animal subject. As referred to herein, "administering" refers to any method by which a composition is applied on or administered to the subject. In various embodiments, the administration is topical, wherein the composition is applied to an external surface of the subject, such as to a surface of the oral cavity (e.g., teeth, gingival, and tongue), to the skin, to the eye, and to the hair. The specific route and method of administration will depend, of course, on the intended use of the composition. In other examples, the functional material may be applied to fabric or dish surfaces by washing with home care products containing films including the functional material.

Disclosed herein are methods for administering a functional material to a human or animal subject in need thereof, comprising topically applying to the subject a composition comprising a carrier comprising a film, wherein the film comprises a functional material as well. In one embodiment, the method additionally comprises disrupting the film after topically applying the film. Such disruption may be accomplished by any of a variety of methods, including chemical and/or mechanical means. Chemical means include degradation of the film by contact with water or a material present at the site of administration (e.g., saliva in an oral care application). Physical means include agitation, grinding, and shear forces produced by application of physical energy to the composition during use (e.g., brushing in a dentifrice application or washing application on the skin which includes the use of a pouf).

Also disclosed herein are methods for the treatment of an oral care condition. As referred to herein, an "oral care condition" is any disorder or condition which can be prevented or treated by administration of a composition to the oral cavity, including disorders or conditions of the teeth, oral mucosa, gingiva and tongue. Such conditions include caries, gingivitis, periodontitis, and cosmetic conditions such as yellowing and malodor.

Also disclosed herein are methods for administering a functional material to a human or animal subject in need thereof, comprising topically applying to the subject a composition comprising a carrier comprising a film in a body wash carrier, wherein the film preferably comprises the functional material. In one embodiment, the method additionally comprises disrupting the film after topically applying the film, e.g., by mechanical means. In various embodiments, the present invention provides methods for the treatment of a dermatological condition.

The films of the present invention, in various embodiments, disintegrate during use of the composition. In other embodiments, the film does not disintegrate during use of the composition. In some embodiments, the film releases a functional material when it disintegrates during use. As referred to herein, "disintegrate" refers to physical disruption of the film or fragment material, so as to produce a film or film pieces of reduced size compared to the original film. Such disruption may be through mechanical, thermal, chemical, or physical-chemical means. The disintegration can result, for example, from shearing, dissolution, grinding, or exposure to elevated temperatures during use.

The functional material that may be released when the film disintegrates may provide enhanced performance of the aqueous composition during use. In some embodiments, the film is substantially insoluble but breakable in water by being dispersible, i.e., it breaks down into small pieces, for example, as a result of shearing by application of shear mechanical force. In some embodiments, a polymer is insoluble but swellable, such as when the film contains a sufficient amount of water-insoluble polymer to swell but not dissolve within 10 minutes when placed in water. The dissolution can occur as a result of, for example, shearing and/or exposure to a solvent comprising a high concentration of water, such as saliva, or by dilution in water, and in washing skin or preparing a cleansing solution.

Normal use would generally be either by hand or utilizing an implement such a brush, pad, pouf or washcloth. The greater the level of the hydrophilic film components, the less stable the film will be in the formulation but the less shear it will need during use to break down. Preferably, the film disintegrates upon usage within 1-60 seconds, more preferable 5-50 seconds, even more preferable 20-30 seconds.

The amount of water-soluble, partially water-soluble, and water-insoluble polymers in the film may be balanced so that the rate of release of the functional material (and disintegration of the film) is dependent on how long the product is used and/or how much shear force is applied during use of the product. For example, a film in hand soap may be formulated so that during a consumer's regular hand washing routine it completely disintegrates. In a body wash composition used with a pouf, the film may be formulated to disintegrate immediately upon rubbing to the skin or it may be formulated to disintegrate slowly to provide tactile or visual awareness of the film's release of functional material to the user. Normal use would generally be either by hand or utilizing an implement such a pouf or washcloth. The greater the level of the hydrophilic film components, the less stable the film will be in the formulation but the less shear it will need during use to break down. Preferably, the film disintegrates upon usage within 1-60 seconds, more preferable 5-50 seconds, even more preferable 20-30 seconds The same will apply for hair, oral care, and home care compositions of the invention containing films.

<u>Examples</u>

The invention is illustrated by the following examples. The examples are merely illustrative and do not in any way limit the scope of the invention as described and claimed. All parts are parts by weight unless otherwise noted. Various names of chemical components include those listed in the CTFA International Cosmetic Ingredient Dictionary (Cosmetics, Toiletry and Fragrance Association, Inc., 7th cd. 1997). All examples are prophetic.

<u>Example 1 - Body Wash Containing Film Flakes</u>

A suitable container is charged with 44.6 parts by weight deionized water. CARBOPOL AQUA SF-1 (30% solids, available from Noveon), 8.95 parts by weight, is added with stirring. Then, 37.13 parts by weight of aqueous sodium laureth sulfate (25.5% by weight) are added, followed by 0.74 parts by weight of aqueous sodium hydroxide (50% by weight) to bring the pH to about 6.2 to 6.8. Next, 5.64 parts by weight of aqueous cocoamidopropyl betaine (30% by weight) was added. After mixing. 0.4 parts by weight DMDM hydantoin, 0.211 parts by weight aqueous EDTA (39% by weight), and 0.3-1.2 parts by weight of a perfume are added to complete the body wash carrier.

A film is prepared by mixing together 12 parts of a PVP homopolymer (30% solution), 10 parts of a PVP/VA polymer (in a 30% solution) and 40 parts by weight ethanol. To this is added 35 parts of a (30% by weight ethyl methylmethacrylate polymer dispersion and 3 parts of a functional material.

The slurry is drawn down onto a non-stick surface, and dried. The resultant film has a thickness of approximately 0.3 mm. It is cut into non-uniform pieces. Then, 0.6 parts by weight of the film pieces are mixed into the body wash carrier to form the body wash with film flakes.

Example 2 - Preparation of a Dentifrice Containing Film Flakes

A film is prepared as in Example 1. The film pieces are incorporated into a standard tooth cleaning gel formulation in an amount of about 0.2% by weight.

**Claims**

1. A composition comprising a film entrained in a carrier, wherein the film comprises a first polymer having a first solubility and a second polymer having a second solubility, wherein the first solubility is greater than the second solubility in water, the first polymer being water-soluble and the second polymer being water-insoluble; wherein the water-soluble polymer is a combination of poly(vinyl pyrrolidone) and water-soluble poly(vinyl pyrrolidone/vinyl acetate) copolymer, and the water-insoluble polymer comprises one or more polyacrylate copolymers

2. The composition according to claim 1, wherein the carrier has a yield value of 3 to 20 Pascal and a viscosity of 2,000 to 20,000 mPas (centipoise).

3. The composition according to claim 1, wherein the carrier is thixotropic and has a viscosity of at least 4,000 mPas (centipoise).

4. The composition according to claim 1, wherein the film further comprises a functional material selected from the group consisting of therapeutic active materials, flavorants, colorants, pearlescent materials, beads, cosmetic materials, fragrances, and combinations thereof.

**Patentansprüche**

1. Zusammensetzung, umfassend einen Film, der in einem Träger eingeschlossen ist, wobei der Film ein erstes Polymer mit einer ersten Löslichkeit und ein zweites Polymer mit einer zweiten Löslichkeit umfasst, wobei die erste Löslichkeit größer ist als die zweite Löslichkeit in Wasser, wobei das erste Polymer wasserlöslich und das zweite Polymer wasserunlöslich ist; wobei das wasserlösliche Polymer eine Kombination von Poly(vinylpyrrolidon) und wasserlöslichem Poly(vinylpyrrolidon/Vinylacetat)-Copolymer ist und das wasserunlösliche Polymer ein oder mehrere Polyacrylat-Copolymere umfasst.

2. Die Zusammensetzung gemäß Anspruch 1, wobei der Träger einen Yield-Value von 3 bis 20 Pascal und eine Viskosität von 2.000 bis 20.000 mPas (Centipoise) aufweist.

3. Die Zusammensetzung gemäß Anspruch 1, wobei der Träger thixotrop ist und eine Viskosität von mindestens 4.000 mPas (Centipoise) aufweist.

4. Die Zusammensetzung gemäß Anspruch 1, wobei der Film weiterhin ein funktionales Material umfasst, ausgewählt aus der Gruppe bestehend aus therapeutisch wirksamen Materialien, Aromastoffen, Farbstoffen, Perleffektmaterialien, Kügelchen, kosmetischen Materialien, Duftstoffen und Kombinationen davon.

**Revendications**

1. Composition comprenant un film entrainé dans un support, dans laquelle le film comprend un premier polymère ayant une première solubilité et un deuxième polymère ayant une deuxième solubilité, dans laquelle la première solubilité est supérieure à la deuxième solubilité dans l'eau, le premier polymère étant soluble dans l'eau et le deuxième polymère étant insoluble dans l'eau ; dans laquelle le polymère soluble dans l'eau est une combinaison de poly(vinylpyrrolidone) et de copolymère de poly(vinylpyrrolidone/acétate de vinyle) soluble dans l'eau, et le polymère insoluble dans l'eau comprend un ou plusieurs copolymères polyacrylate.

2. Composition selon la revendication 1, dans laquelle le support a une limite apparente d'élasticité de 3 à 20 Pascal et une viscosité de 2 000 à 20 000 mPas (centipoises).

**3.** Composition selon la revendication 1, dans laquelle le support est thixotrope et présente une viscosité d'au moins 4 000 mPas (centipoises).

**4.** Composition selon la revendication 1, dans laquelle le film comprend en outre un matériau fonctionnel choisi dans le groupe constitué par des matières actives thérapeutiques, des agents aromatisants, des colorants, des matières nacrées, des billes, des matières cosmétiques, des parfums et des combinaisons de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6596298 B, Leung
- US 6635702 B, Schmucker-Castner
- US 6503495 B, Alwattari
- US 6352701 B, Scholz

**Non-patent literature cited in the description**

- **MCCLELLAND, D.** Macworld® Photoshop® 4 Bible. IDG Books Worldwide. Inc, 1997, 157-184
- Chemicals Used in Food Processing. National Academy of Sciences, 63-258
- CTFA International Cosmetic Ingredient Dictionary. Cosmetics, Toiletry and Fragrance Association, Inc, 1997